# FASCICULE DE BREVET EUROPEEN

(11) **EP 0 791 351 B1**
(45) Date de publication et mention de la délivrance du brevet: **11.12.2002**
(21) Numéro de dépôt: 97400035.8
(22) Date de dépôt: 08.01.1997
(51) Int. Cl.: A61K 7/11, A61K 7/06

(54) **Composition contenant un polymére fixant, un N-alcane en C5-C8 et de l'acétone**
Zusammensetzung, die ein fixierendes Polymer, ein C5-C8-n-Alkan und Aceton enthält
Composition containing a fixative polymer, an n-C5-C8-alkane and acetone

(30) Priorité: 20.02.1996 FR 9602081
(43) Date de publication de la demande: 27.08.1997
(73) Titulaire: L'OREAL, 75008 Paris (FR)
(72) Inventeur: Adao, Adrien, 75020 Paris (FR)
(74) Mandataire: Andral, Christophe André Louis

(56) Documents cités:
- EP-A- 0 257 444
- EP-A- 0 638 306
- WO-A-93/18735

## Description

L'invention concerne une composition cosmétique, caractérisée par le fait qu'elle comprend (a) un polymère fixant, (b) de l'acétone, (c) un alcool en C₁-C₄, (d) un hydrocarbure linéaire en C₅-C₈ et (e) de l'eau, ainsi qu'un procédé de traitement cosmétique des matières kératiniques à l'aide de ces compositions.

Les compositions capillaires à pulvériser sur les cheveux sont essentiellement constituées d'une solution le plus souvent alcoolique et d'un polymère en mélange éventuellement avec divers adjuvants cosmétiques. Cette solution est conditionnée soit dans un flacon pompe, soit dans un récipient aérosol approprié qui est mis sous pression à l'aide d'un gaz propulseur.

Pour des raisons essentiellement écologiques, on cherche à réduire les composés volatils à la pression atmosphérique, dits COV (Composés Organiques Volatils) qui sont présents dans les compositions. Les COV sont principalement les propulseurs et certains solvants tels que l'éthanol.

Ainsi, la législation de certains pays a imposé un taux maximum de COV dans les compositions cosmétiques en aérosol. Pour diminuer la quantité de COV, on a essayé de remplacer les solvants tels que l'éthanol par de l'eau.

Ces modifications peuvent entraîner des problèmes en particulier sur la qualité de la pulvérisation du spray. Les particules liquides pulvérisées ne sont pas fines, le spray est souvent pincé, c'est à dire non diffus, et la formation d'une légère mousse et/ou de bulles peut se produire.
La pulvérisation étant un élément essentiel pour la qualité finale d'une composition à pulvériser sur les cheveux, il est primordial de remédier à ces inconvénients pour obtenir une bonne répartition sur l'ensemble de la chevelure et une fixation et/ou un conditionnement correct des cheveux.
D'autre part, les compositions contenant de l'eau présentent l'inconvénient de sécher trop lentement et de conduire à l'effondrement de la coiffure. On a donc été amené à rechercher des compositions avec une vitesse de séchage plus rapide.

L'invention a donc pour but des compositions aqueuses susceptibles de conduire à des propriétés analogues à celles des compositions à forte teneur en alcool à savoir une bonne diffusion lors de l'application, un bon pouvoir de fixation, une résistance à l'humidité, une bonne élimination au shampooing et au brossage et une bonne vitesse de séchage.

La demanderesse vient maintenant de découvrir, de façon inattendue et surprenante, qu'en associant les polymères habituellement utilisés dans les compositions de maintien de la coiffure avec un hydrocarbure linéaire en C₅-C₈, de l'acétone, un alcool et de l'eau, il est possible d'obtenir des compositions présentant un temps de séchage amélioré.

L'invention a donc pour objet une composition cosmétique, caractérisée par le fait qu'elle comprend (a) un polymère fixant, (b) de l'acétone, (c) un alcool en C₁-C₄, (d) un hydrocarbure linéaire en C₅-C₈ et (e) de l'eau.

Les compositions présentent l'avantage d'avoir une excellente pulvérisation et/ou vaporisation. Les gouttelettes des sprays sont très fines, la répartition des compositions sur la chevelure est excellente.

La demanderesse a constaté également que les compositions conformes à l'invention ne conduisaient pas à des phénomènes de mousse sur les cheveux entraînant un effet blanchissant ni à des phénomènes de poudrage ou de poissage. Elles apportent en outre de bonnes propriétés de coiffage et de recoiffage, une bonne tenue à la chevelure ainsi qu'un démêlage facile.

Dans le cadre de la présente demande, on entend par compositions cosmétiques pour le maintien de la coiffure toute composition ayant pour fonction de fixer temporairement la forme de la coiffure, comme par exemple les laques, les compositions de mise en plis et sprays de coiffage. Par pouvoir fixant de la composition, on désigne l'aptitude de cette dernière à donner aux cheveux une cohésion de telle sorte que la mise en forme initiale de la coiffure soit conservée. Par polymère fixant, on entend tout polymère ayant pour fonction de fixer temporairement la forme de la coiffure.

Dans les compositions selon l'invention, l'acétone et l'hydrocarbure linéaire en C₅-C₈ sont présents généralement dans un rapport pondéral acétone/hydrocarbure linéaire en C₅-C₈ supérieur à 0,25 et de préférence compris entre 0,25 et 20.

Dans les compositions selon l'invention, la concentration en alcool est généralement comprise entre 3 et 80 % en poids, et de préférence entre 5 et 40 % en poids, par rapport au poids total de la composition.

La concentration en hydrocarbure linéaire en C₅-C₈ est comprise généralement entre 1 et 50 % en poids, et de préférence entre 3 et 25 % en poids, par rapport au poids total de la composition.

La concentration en acétone est généralement comprise entre 5 et 80% en poids, et de préférence entre 20 et 50% en poids, par rapport au poids total de la composition.

Le polymère fixant est généralement présent dans des concentrations comprises entre 0,05 et 30%, et de préférence dans des concentrations comprises entre 0,5% et 15 %, en poids par rapport au poids total de la composition.

Les alcools en C₁-C₄, qui peuvent être utilisés seuls ou en mélange, sont par exemple l'éthanol, l'isopropanol, les polyalcools tels que le diéthylèneglycol, les monoalkyléthers de glycol, de diéthylèneglycol ou de propylèneglycol. L'éthanol est particulièrement préféré.

Les hydrocarbures linéaires en C₅-C₈ sont par exemple les n-alcanes en C₅-C₈ : le n-pentane, le n-hexane, le n-heptane et le n-octane. Le n-pentane est particulièrement préféré selon l'invention.

Selon l'invention, on peut utiliser tout polymère fixant connu en soi. On peut utiliser en particulier un polymère fixant choisi parmi les polymères anioniques, cationiques, amphotères, non ioniques et leurs mélanges.

Les polymères fixants peuvent être utilisés sous forme solubilisée ou sous forme de latex ou de pseudolatex (dispersions aqueuses de particules solides de polymère).

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 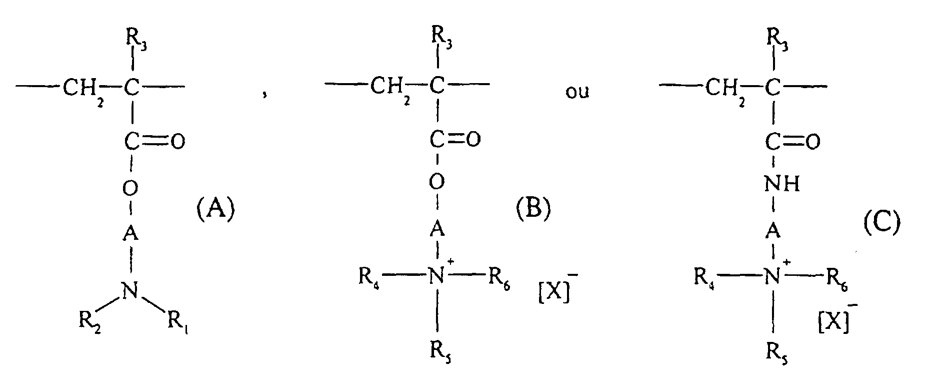 dans lesquelles:
   R₃ désigne un atome d'hydrogène ou un radical CH₃;
   A est un groupe alkyle linéaire ou ramifié de 1 à atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle;
   R₁ et R₂ représentent hydrogène ou un groupe alkyle ayant de 1 à 6 atomes de carbone;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs, des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/vinylpyrrolidone tel que le produit vendu sous la dénomination GAFFIX VC 713 par la société ISP,
   - et les copolymères vinylpyrrolidone / méthacrylamide de diméthylaminopropyle quaternisé tel que le produit vendu sous la dénomination "GAFQUAT HS 100" par la société ISP.
(2) les polysaccharides quaternisés décrits plus particulièrement dans les brevets américains 3.589.578 et 4.031.307 tel que le produit commercialisé sous la dénomination JAGUAR C 13 S par la société MEYHALL;
(3) les copolymères quaternaires de vinylpyrrolidone et de vinylimidazole;
(4) les chitosanes ou leurs sels;
les sels utilisables sont en particulier les acétate, lactate, glutamate, gluconate ou le pyrrolidone carboxylate de chitosane .
Parmi ces composés, on peut citer le chitosane ayant un taux de désacétylation de 90,5% vendu sous la dénomination KYTAN BRUT STANDARD par la société ABER TECHNOLOGIES, le pyrrolidone carboxylate de chitosane vendu sous la dénomination KYTAMER PC par la société AMERCHOL .

Les polymères fixants anioniques généralement utilisés sont des polymères comportant des groupements dérivés d'acide carboxylique, sulfonique ou phosphorique et ont un poids moléculaire compris entre environ 500 et 5.000.000.
1) Les groupements carboxyliques sont apportés par des monomères mono ou diacides carboxyliques insaturés tels que ceux répondant à la formule : 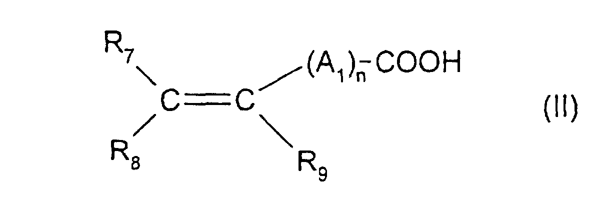
dans laquelle n est un nombre entier de 0 à 10, A₁ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ; Dans la formule précitée un radical alkyle inférieur désigne de préférence un groupement ayant 1 à 4 atomes de carbone et en particulier, méthyle et éthyle.

Les polymères fixants anioniques à groupements carboxyliques préférés selon l'invention sont :
A) les homo- ou copolymères d'acide acrylique ou méthacrylique ou leurs sels et en particulier les produits vendus sous les dénominations VERSICOL E ou K par la société ALLIED COLLOID et ULTRAHOLD par la société BASF. Les copolymères d'acide acrylique et d'acrylamide vendus par exemple sous la forme de leur sel de sodium sous les dénominations RETEN 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀ par exemple de lauryle tel que celui vendu par la société ISP sous la dénomination ACRYLIDONE LM et les terpolymères acide méthacrylique/ acrylate d'éthyle/ acrylate de tertiobutyle tel que le produit vendu sous la dénomination LUVIMER 100 P par la société BASF.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
D) les copolymères dérivés d'acides ou d'anhydrides carboxyliques monoinsaturés en C4-C8 choisis parmi :
   - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. ; De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
   - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupement acrylamide, méthacrylamide, α-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
   Ces polymères sont par exemple décrits dans les brevets français 2.350.384 et 2.357.241 de la demanderesse.
E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères fixants anioniques sont de préférence choisis parmi les copolymères d'acide acrylique tels que le terpolymère acide acrylique / acrylate d'éthyle / N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle / néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, les copolymères d'acide méthacrylique et d'acrylate d'éthyle vendu sous la dénomination LUVIMER MAEX ou MAE par la société BASF et les copolymères acétate de vinyle/acide crotonique vendu sous la dénomination LUVISET CA 66 par la société BASF, les terpolymères de vinylpyrrolidone, d'acide acrylique et de méthacrylate d'alkyle en C₁-C₂₀ par exemple de lauryle tel que celui vendu par la société ISP sous la dénomination ACRYLIDONE LM et les copolymères acétate de vinyle/acide crotonique greffé par du polyéthylèneglycol sous la dénomination ARISTOFLEX A par la société BASF.

Les polymères fixants anioniques les plus particulièrement préférés sont choisis parmi le copolymère méthylvinyléther / anhydride maléique mono estérifié vendu sous la dénomination GANTREZ ES 425 par la société ISP, le terpolymère acide acrylique /acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX OU MAE par la société BASF, le terpolymère de vinylpyrrolidone / acide acrylique/méthacrylate de lauryle vendu sous la dénomination ACRYLIDONE LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène a,b-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atome de carbone des acides ou des anhydrides maléïque ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER, AMPHOMER LV71 ou LOVOCRYL 47 par la société NATIONAL STARCH.
(3) les polyamino amides réticulés et alcoylés partiellement ou totalement dérivant de poyaminoamides de formule générale : 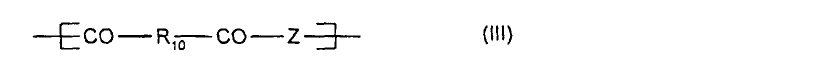 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 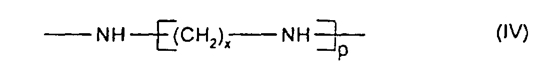 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (IV) ci-dessus, dans lequel x=2 et p=1 et qui dérive de l'éthylènediamine, ou le radical dérivant de la pipérazine : 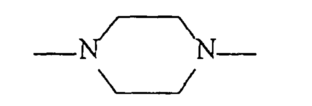
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
   (4) les polymères comportant des motifs zwittérioniques de formule : 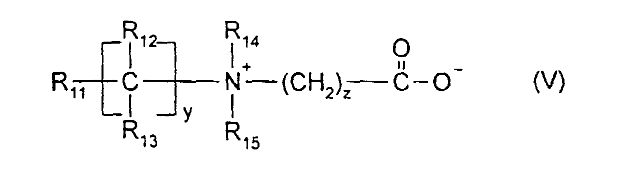 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.
      Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
      A titre d'exemple, on peut citer le copolymère de méthacrylate de méthyle / diméthyl carboxyméthylammonio éthylméthacrylate de méthyle tel que le produit vendu sous la dénomination DIAFORMER Z301 par la société SANDOZ.
   (5) les polymères dérivés du chitosane comportant des motifs monomères répondant aux formules suivantes : 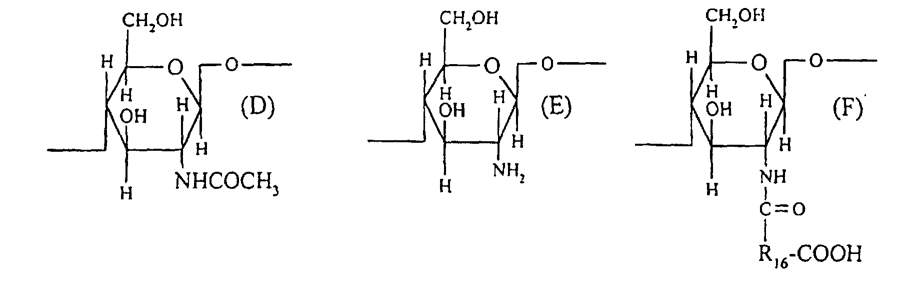 le motif D étant présent dans des proportions comprises entre 0 et 30%, le motif E dans des proportions comprises entre 5 et 50% et le motif F dans des proportions comprises entre 30 et 90%, étant entendu- que dans ce motif F, R₁₆ représente un radical de formule 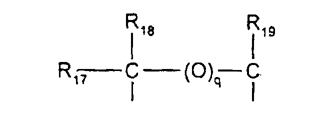 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, acoylthio, sulfonique, un reste alcoylthio dont le groupe alcoyle porte un reste amino, l'un au moins des radicaux R₁₇, R₁₈ et R₁₉ étant dans ce cas un atome d'hydrogène ; ou si q=1, R₁₇, R₁₈ et R₁₉ représentent chacun un atome d'hydrogène, ainsi que les sels formés par ces composés avec des bases ou des acides.
   (6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
   (7) Les polymères répondant à la formule générale (VI) sont par exemple décrits dans le brevet français 1 400 366 : 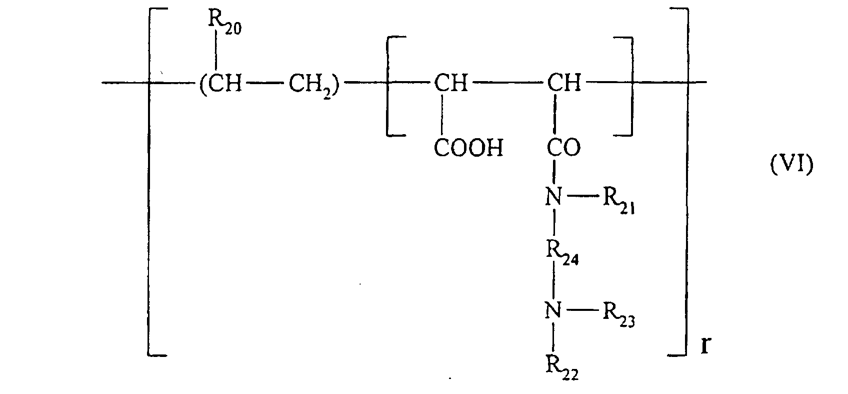 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂- , -CH₂-CH₂-CH₂- , -CH₂-CH(CH₃)- , R₂₂ ayant les significations mentionnées ci-dessus,
      ainsi que les homologues supérieurs de ces radicaux et contenant jusqu'à 6 atomes de carbone.
   (8) Des polymères amphotères du type -D-X-D-X choisis parmi:
      a) les polymères obtenus par action de l'acide chloracétique ou le chloracétate de sodium sur les composés comportant au moins un motif de formule :

         -D-X-D-X-D- (VII)

         où D désigne un radical 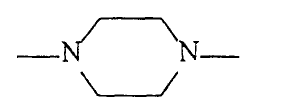 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
      b) Les polymères de formule :

         -D-X-D-X- (VII')
      où D désigne un radical 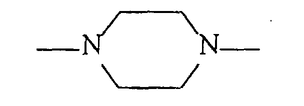 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
   (9) les copolymères alkyl(C1-C5)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/butylaminoethylmethacrylate copolymer tels que les produits vendus sous les dénominations AMPHOMER, AMHOMER LV 71 ou LOVOCRYL 47 par la société NATIONAL STARCH et ceux de la famille (4) tels que les copolymère de méthacrylate de méthyle / diméthyl carboxyméthylammonio éthylméthacrylate de méthyle par exemple vendu sous la dénomination DIAFORMER Z301 par la société SANDOZ.

Les polymères fixants non ioniques utilisables selon la présente invention sont choisis par exemple parmi :
- les poly β alanines décrites plus particulièrement dans le brevet français n°2508795 ;
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les homopolymères de chlorure de vinyle tels que les produits proposés sous les noms de GEON 460X45, GEON 460X46 et GEON 577 par la société GOODRICH ;
- les cires de polyéthylène tels que les produits proposés sous les dénominations AQUACER 513 et AQUACER 533 par la société BYK CERA ;
- les cires de polyéthylène/polytétrafluoroéthylène tels que les produits proposés sous les dénominations DREWAX D-3750 par la société DREW AMEROID et WAX DISPERSION WD-1077 par la société R.T. NEWEY ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS
- les homopolymères de styrène tels que le produit RHODOPAS 5051 proposé par la société RHONE POULENC ;
- les copolymères de styrène et de (méth)acrylate d'alkyle tels que les produits MOWILITH LDM 6911, MOWILITH DM 611 et MOWILITH LDM 6070 proposés par la société HOECHST, les produits RHODOPAS SD 215 et RHODOPAS DS 910 proposés par la société RHONE POULENC;
- les copolymères de styrène, de méthacrylate d'alkyle et d'acrylate d'alkyle tels que le produit DAITISOL SPA proposé par la société WACKHERR ;
- les copolymères de styrène et de butadiène tels que les produits RHODOPAS SB 153 et RHODOPAS SB 012 proposés par la société RHONE POULENC ;
- les copolymères de styrène, de butadiène et de vinylpyridine tels que les produits GOODRITE SB VINYLPYRIDINE 2528X10 et GOODRITE SB VINYLPYRIDINE 2508 proposés par la société GOODRICH ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.
Les radicaux alkyle des polymères non ioniques ont de 1 à 6 atomes de carbone sauf mention contraire.

Selon l'invention, on peut également utiliser les polymères fixants de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule 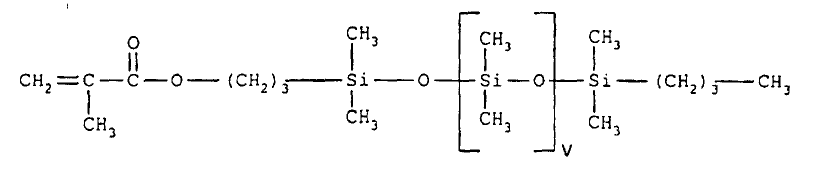 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon la présente invention, les polymères fixants sont de préférence des polymères anioniques.

Les compositions selon l'invention contiennent de préférence de 5 à 50% d'eau par rapport au poids total de la composition et plus particulièrement de 5 à 30% d'eau.

Le pH des compositions selon l'invention est généralement compris entre 2 et 9, et en particulier entre 3 et 8,5. Il peut être ajusté à la valeur choisie au moyen d'agents alcalinisants ou acidifiants habituellement utilisés en cosmétique pour ce type d'application.

Lorsque la composition selon l'invention est pressurisée sous forme d'aérosol, l'aérosol comprend la composition décrite ci-dessus, appelée jus, et au moins un agent propulseur qui peut être choisi parmi les hydrocarbures volatils tels que le n-butane, le propane, l'isobutane, les hydrocarbures chlorés et/ou fluorés et leurs mélanges. On peut également utiliser en tant qu'agent propulseur le gaz carbonique, le protoxyde d'azote, le diméthyléther, l'azote, l'air comprimé et leurs mélanges. Le jus et le propulseur peuvent se trouver dans des compartiments différents dans le récipient aérosol.

Selon l'invention, la concentration en propulseur(s) est généralement comprise entre 5 et 90% en poids par rapport au poids total de la composition pressurisée et de préférence entre 15 et 50% en poids.

Les compositions selon l'invention pressurisée ou non peuvent encore contenir des agents tensioactifs, des agents conservateurs, des séquestrants, des adoucissants, des colorants, des agents modificateurs de viscosité, des agents anti-mousse, des agents hydratants, des agents antipelliculaires, des agents anti-séborrhéiques, des filtres solaires, des protéines, des vitamines, des plastifiants, des hydroxyacides, des électrolytes et des parfums.

Les compositions selon l'invention peuvent également contenir des agents conditionneurs. Ceux-ci peuvent alors être choisis par exemple parmi les huiles et les cires naturelles ou synthétiques, les alcools gras, les esters d'alcools polyhydriques, les glycérides, les huiles, les gommes et les résines de silicone ou les mélanges de ces différents composés.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions utilisées selon l'invention sont par exemple des compositions capillaires non rincées.

Elles sont plus particulièrement des lotions de mise en plis, des lotions pour le brushing, des compositions de fixation (laques) et de coiffage. Les lotions peuvent être conditionnées sous diverses formes notamment dans des vaporisateurs, des flacons pompe ou dans des récipients aérosols afin d'assurer une application de la composition sous forme vaporisée.

L'invention a encore pour objet un procédé de traitement cosmétique des matières kératiniques, telles que les cheveux, caractérisé en ce qu'il consiste à appliquer sur matières kératiniques une composition cosmétique telle que définie précédemment.

La préparation des compositions selon l'invention est réalisée selon des méthodes bien connues de l'état de la technique. En particulier, les ingrédients sont mélangés puis conditionnés dans un récipient approprié selon l'utilisation envisagée. Lorsque la composition est pressurisée en aérosol, le jus est placé dans un récipient qui est ensuite scellé par un système de valve, le propulseur est alors introduit dans le récipient.

Les exemples qui suivent servent à illustrer l'invention sans toutefois présenter un caractère limitatif.

### EXEMPLE 1

On a préparé un spray de fixation capillaire conditionné dans un flacon pompe de composition suivante :
- Terpolymère acide acrylique / acrylate d'éthyle / N-tertiobutylacrylamide vendu sous la dénomination ULTRAHOLD STRONG par la société BASF 5 g
- Amino-2 méthyl-2 propanol qsp neutralisation à 100% du copolymère
- Eau 20 g
- Acétone 35 g
- n-pentane 5 g
- Ethanol qsp 100 g

On a appliqué cette composition sur une chevelure, la diffusion de la composition est bonne, les particules sont fines. La composition sèche rapidement et ainsi la coiffure est fixée dans la forme désirée.

### EXEMPLE 2

On a préparé un spray de fixation capillaire (A) conditionné dans un flacon aérosol de composition suivante :
- Terpolymère acétate de vinyle / tertio-butyl benzoate de vinyle / acide crotonique 4 g
- Amino-2 méthyl-2 propanol qsp neutralisation à 100% du copolymère
- Eau 10 g
- Acétone 35 g
- n-pentane 10 g
- Diméthyléther 35 g
- Ethanol qsp 100 g

On a appliqué cette composition sur une chevelure, la diffusion de la composition est bonne, les particules sont fines. La composition sèche rapidement et ainsi la coiffure est fixée dans la forme désirée.

On a préparé un spray de fixation capillaire (B) non conforme à l'invention conditionné dans un flacon aérosol de composition suivante :
- Terpolymère acétate de vinyle / tertio-butyl benzoate de vinyle / acide crotonique 4 g
- Amino-2 méthyl-2 propanol qsp neutralisation à 100% du copolymère
- Eau 10 g
- Acétone 35 g
- Diméthyléther 45 g
- Ethanol qsp 100 g

Cette composition ne contient pas de n-pentane.

Dans deux récipients identiques, on a pulvérisé dans l'un la composition (A) et dans l'autre la même quantité de composition (B). Les deux récipients sont maintenus à 20°C. On pèse la quantité de produit restant dans le récipient au cours du temps.

Les résultats sont rassemblés dans le tableau suivant :

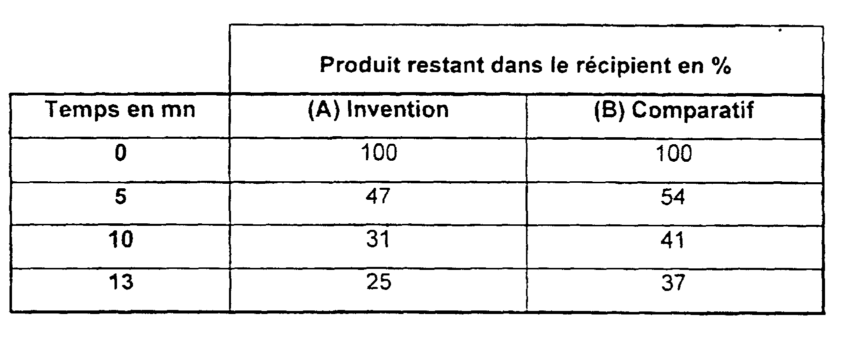

La composition (A) sèche plus vite que la composition (B), puisqu'au bout de 13 minutes, il ne reste que 25% de la composition (A) au lieu de 37% en poids de la composition (B).

## Revendications

1. Composition cosmétique, comprenant (a) un polymère fixant, (b) de l'acétone, (c) un alcool en C₁-C₄, (d) un hydrocarbure linéaire en C₅-C₈ et (e) de l'eau, **caractérisée par le fait que** la concentration en alcool est comprise entre 5 et 40 % en poids par rapport au poids total de la composition.

2. Composition selon la revendication 1, **caractérisée en ce que** l'acétone et l'hydrocarbure linéaire en C₅-C₈ sont présents dans un rapport acétone/hydrocarbure linéaire en C₅-C₈ supérieur à 0,25.

3. Composition selon la revendication précédente, **caractérisée en ce que** le rapport acétone/ hydrocarbure linéaire en C₅-C₈ est compris entre 0,25 et 20.

4. Composition selon l'une quelconque des revendications précédentes, **caractérisée en ce que** le polymère fixant est présent dans des concentrations comprises entre 0,05 et 30% en poids par rapport au poids total de la composition.

5. Composition selon la revendication précédente, **caractérisée en ce que** le polymère fixant est présent dans des concentrations comprises entre 0,5% et 15 % en poids par rapport au poids total de la composition.

6. Composition selon l'une quelconque des revendications précédentes, **caractérisée en ce que** la concentration en hydrocarbure linéaire en C₅-C₈ est comprise entre 1 et 50 % en poids par rapport au poids total de la composition.

7. Composition selon ta revendication précédente, **caractérisée en ce que** la concentration en hydrocarbure linéaire en C₅-C₈ est comprise entre 3 et 25 % en poids par rapport au poids total de la composition.

8. Composition selon l'une quelconque des revendications précédentes, **caractérisée en ce que** la concentration en acétone est comprise entre 5 et 80% en poids et de préférence entre 20 et 50% en poids par rapport au poids total de la composition.

9. Composition selon l'une quelconque des revendications précédentes, **caractérisée en ce que** les hydrocarbures linéaires en C₅-C₈ sont les n-alcanes en C₅-C₈.

10. Composition selon la revendication précédente, **caractérisée en ce que** l'hydrocarbure linéaire en C₅-C₈ est le n-pentane.

11. Composition selon l'une quelconque des revendications précédentes, **caractérisée en ce que** le polymère fixant est choisi parmi les polymères anioniques, cationiques, amphotères, zwittérioniques, non ioniques et leurs mélanges.

12. Composition selon la revendication précédente, **caractérisée par** leₒ fait que le polymère fixant anionique est choisi parmi :
- les polymères comportant des motifs carboxyliques dérivant de monomères mono ou diacides carboxyliques insaturés de formule 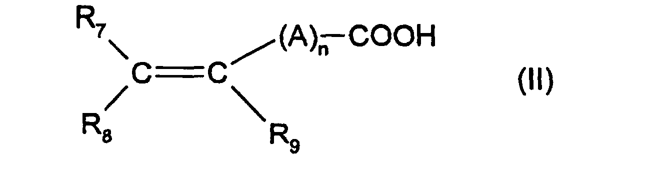 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;
- les polymères comprenant des motifs dérivant d'acide sulfonique tels que des motifs vinylsulfonique, styrènesulfonique, acrylamido alkylsulfonique.

13. Composition selon la revendication précédente, **caractérisée en ce que** le polymère fixant anionique est choisi parmi
A) les homo- ou copolymères d'acide acrylique ou méthacrylique ou leurs sels, les copolymères d'acide acrylique et d'acrylamide et leurs sels, les sels de sodium d'acides polyhydroxycarboxyliques ;
B) les copolymères des acides acrylique ou méthacryliques avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés ; les copolymères de ce type comportant dans leur chaîne un motif acrylamide éventuellement N-alkylé et/ou hydroxyalkylé, les copolymères d'acide acrylique et de méthacrylate d'alkyle en C₁-C₄ ;
C) les copolymères dérivés d'acide crotonique tels que ceux comportant dans leur chaîne des motif acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carboné, ces polymères pouvant éventuellement être greffés et réticulés ;
D) les copolymères dérivés d'acides ou d'anhydrides carboxyliques monoinsaturés en C4-C8 choisis parmi :
- les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. ;
- les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupement acrylamide, méthacrylamide, α-oléfine, esters acryliques ou méthacryliques, acides acrylique ou méthacrylique ou vinylpyrrolidone dans leur chaîne,
les fonctions anhydrides de ces copolymères étant éventuellement monoestérifiées ou monoamidifiées.
E) tes polyacrylamides comportant des groupements carboxylates.

14. Composition selon la revendication précédente, **caractérisée en ce que** le polymère fixant anionique est choisi parmi :
- les copolymères d'acide acrylique tels que le terpolymère acide acrylique/acrylate d'éthyle/N-tertiobutylacrylamide ;
- les copolymères dérivés d'acide crotonique tels que les terpolymères acétate de vinyle / tertio-butyl benzoate de vinyle / acide crotonique et les terpolymères acide crotonique/acétate de vinyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié ;
- les copolymères d'acide méthacrylique et de méthacrylate de méthyle ;
- les copolymères d'acide méthacrylique et d'acrylate d'éthyle;
- les copolymères acétate de vinyle/acide crotonique ;
- les terpolymères acétate de vinyle/acide crotonique/polyéthylèneglycol.

15. Composition selon la revendication 11, **caractérisée en ce que** le polymère fixant amphotère est choisi parmi les polymères comportant des motifs dérivant:
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) au moins un comonomère basique tel que des esters à substituants amine primaire, secondaire, tertiaire et quaternaire des acides acrylique et méthacrylique et le produit de quatemisation du méthacrylate de diméthylaminoéthyle avec le sulfate de diméthyle ou diéthyle.

16. Composition selon la revendication précédente, **caractérisée en ce que** le polymère fixant amphotère est choisi parmi les copolymères dont la dénomination CTFA est Octylacrylamide/ acrylates/ butylaminoethylmethacrylate copolymer et les copolymères de méthacrylate de méthyle / diméthyl carboxyméthylammonio éthylméthacrylate de méthyle.

17. Composition selon la revendication 11, **caractérisé par le fait que** le polymère fixant non ionique est choisi parmi :
- les poly β alanines ;
- les polyalkyloxazoline ;
- les homopolymères d'acétate de vinyle ;
- les copolymères d'acétate de vinyle et d'ester acrylique ;
- les copolymères d'acétate de vinyle et d'éthylène ;
- les copolymères d'acétate de vinyle et d'ester maléïque ;
- les homopolymères de chlorure de vinyle ;
- les cires de polyéthylène ;
- les cires de polyéthylène/polytétrafluoroèthylène ;
- les copolymères de polyéthylène et d'anhydride maléïque ;
- les homopolymères d'acrylates d'alkyle et les homopolymères de méthacrylates d'alkyle ;
- les copolymères d'esters acryliques tels que par exemple les copolymères d'acrylates d'alkyle et de méthacrylates d'alkyles ;
- les copolymères d'acrylonitrile et d'un monomère non ionique choisi par exemple parmi le butadiène et les (méth)acrylates d'alkyle ;
- les homopolymères de styrène ;
- les copolymères de styrène et de (méth)acrylate d'alkyle ;
- les copolymères de styrène, de méthacrylate d'alkyle et d'acrylate d'alkyle ;
- les copolymères de styrène et de butadiène ;
- les copolymères de styrène, de butadiène et de vinylpyridine ;
- les copolymères d'acrylate d'alkyle et d'uréthanne.

18. Composition selon la revendication 11, **caractérisée en ce que** le polymère fixant cationique est choisi parmi :
- le copolymère d'acrylamide et de diméthylaminoéthyl méthacrylate quatemisé au sulfate de diméthyle,
- les copolymères d'acrylamide et de chlorure de méthacryloyloxyéthyltriméthylammonium,
- le copolymère d'acrylamide et de méthosulfate de méthacryloyloxyéthyltriméthylammonium,
- les copolymères vinylpyrrolidone / acrylate ou méthacrylate de dialkylaminoalkyle quatemisés ou non,
- les terpolymère méthacrylate de diméthyl amino éthyle/ vinylcaprolactame/vinylpyrrolidone,
- et le copolymère vinylpyrrolidone / méthacrylamide de diméthylaminopropyle quaternisé.

19. Composition pressurisée en aérosol **caractérisée par le fait qu'**elle comprend une composition telle que décrite à l'une quelconque des revendications précédentes et au moins un agent propulseur.

20. Composition selon la revendication précédente, **caractérisée par le fait que** l'agent propulseur est choisi parmi les hydrocarbures volatils tels que le n-butane, le propane, l'isobutane, les hydrocarbures chlorés et/ou fluorés, le gaz carbonique, le protoxyde d'azote, le diméthyléther, l'azote, l'air comprimé et leurs mélanges.

21. Composition selon l'une quelconque des revendications 19 ou 20, **caractérisée par** le fait l'agent propulseur est présent dans des concentrations comprises entre 5 et 90% en poids par rapport au poids total de la composition pressurisée.

22. Composition selon la revendication précédente, **caractérisée par** le fait l'agent propulseur est présent dans des concentrations comprises entre 15 et 50% en poids par rapport au poids total de la composition pressurisée.

23. Procédé de traitement cosmétique des matières kératiniques, telles que les cheveux, **caractérisé en ce qu'**il consiste à appliquer sur les matières kératiniques une composition cosmétique telle que définie dans l'une quelconque des revendications 1 à 22.

24. Utilisation de la composition telle que définie dans l'une quelconque des revendications 1 à 22, comme, ou pour la fabrication, de lotion de mise en plis, lotion pour le brushing, composition de coiffage ou de fixation des cheveux.

## Patentansprüche

1. Kosmetische Zusammensetzung, die (a) ein fixierendes Polymer, (b) Aceton, (c) einen C₁₋₄-Alkohol, (d) einen geradkettigen C₅₋₈-Kohlenwasserstoff und (e) Wasser enthält, **dadurch gekennzeichnet, daß** die Konzentration des Alkohols im Bereich von 5 bis 40 Gew.-%, bezogen auf das Gesamtgewicht der Zusammensetzung, liegt.

2. Zusammensetzung nach Anspruch 1, **dadurch gekennzeichnet, daß** das Aceton und der geradkettige C₅₋₈-Kohlenwasserstoff in einem Verhältnis Aceton/geradkettiger C₅₋₈-Kohlenwasserstoff über 0,25 vorliegen.

3. Zusammensetzung nach dem vorhergehenden Anspruch, **dadurch gekennzeichnet, daß** das Verhältnis Aceton/geradkettiger C₅₋₈-Kohlenwasserstoff im Bereich von 0,25 bis 20 liegt.

4. Zusammensetzung nach einem der vorhergehenden Ansprüche, **dadurch gekennzeichnet, daß** das fixierende Polymer in Konzentrationen im Bereich von 0,05 bis 30 Gew.-%, bezogen auf das Gesamtgewicht der Zusammensetzung, vorliegt.

5. Zusammensetzung nach dem vorhergehenden Anspruch, **dadurch gekennzeichnet, daß** das fixierende Polymer in Konzentrationen im Bereich von 0,5 bis 15 Gew.-%, bezogen auf das Gesamtgewicht der Zusammensetzung, vorliegt.

6. Zusammensetzung nach einem der vorhergehenden Ansprüche, **dadurch gekennzeichnet, daß** die Konzentrationen des geradkettigen C₅₋₈-Kohlenwasserstoff im Bereich von 1 bis 50 Gew.-%, bezogen auf das Gesamtgewicht der Zusammensetzung, liegt.

7. Zusammensetzung nach dem vorhergehenden Anspruch, **dadurch gekennzeichnet, daß** die Konzentrationen des geradkettigen C₅₋₈-Kohlenwasserstoff im Bereich von 3 bis 25 Gew.-%, bezogen auf das Gesamtgewicht der Zusammensetzung, liegt.

8. Zusammensetzung nach einem der vorhergehenden Ansprüche, **dadurch gekennzeichnet, daß** die Konzentrationen des Aceton im Bereich von 5 bis 80 Gew.-% und vorzugsweise 20 bis 50 Gew.-%, bezogen auf das Gesamtgewicht der Zusammensetzung, liegt.

9. Zusammensetzung nach einem der vorhergehenden Ansprüche, **dadurch gekennzeichnet, daß** die geradkettigen C₅₋₈-Kohlenwasserstoffe C₅₋₈-n-Alkane sind.

10. Zusammensetzung nach dem vorhergehenden Anspruch, **dadurch gekennzeichnet, daß** es sich bei dem geradkettigen C₅₋₈-Kohlenwasserstoff um das n-Pentan handelt.

11. Zusammensetzung nach einem der vorhergehenden Ansprüche, **dadurch gekennzeichnet, daß** das fixierende Polymer unter den anionischen, kationischen, amphoteren, zwitterionischen oder nichtionischen Polymeren und deren Gemischen ausgewählt ist.

12. Zusammensetzung nach dem vorhergehenden Anspruch, **dadurch gekennzeichnet, daß** das anionische fixierende Polymer ausgewählt ist unter:
- den Polymeren, die Carboxyeinheiten aufweisen, die von ungesättigten Mono- oder Dicarbonsäuremonomeren der Formel: 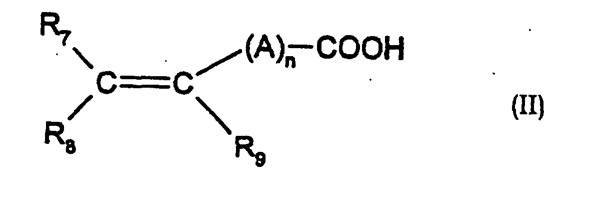 abgeleitet sind, wobei n 0 oder eine ganze Zahl von 1 bis 10 bedeutet, A eine Methylengruppe ist, die gegebenenfalls an das Kohlenstoffatom der ungesättigten Gruppe oder, wenn n größer 1 ist, an die benachbarte Methylengruppe über ein Heteroatom, wie Sauerstoff oder Schwefel, gebunden ist, R₇ Wasserstoff, Phenyl oder Benzyl bedeutet, R₈ Wasserstoff, eine niedere Alkylgruppe oder Carboxy ist und R₉ Wasserstoff, eine niedere Alkylgruppe,
-CH₂-COOH, Phenyl oder Benzyl bedeutet; und
- den Polymeren, die Einheiten enthalten, die von Sulfonsäuren abgeleitet sind, wie Vinylsulfonsäureeinheiten, Styrolsulfonsäureeinheiten und Acrylamidoalkylsulfonsäureeinheiten.

13. Zusammensetzung nach dem vorhergehenden Anspruch, **dadurch gekennzeichnet, daß** das anionische fixierende Polymer ausgewählt ist unter:
A) den Homo- oder Copolymeren von Acrylsäure oder Methacrylsäure oder deren Salzen, Copolymeren von Acrylsäure und Acrylamid und deren Salzen und Natriumsalzen von Polyhydroxycarbonsäuren;
B) den Copolymeren von Acrylsäure oder Methacrylsäure und einem monoethylenisch ungesättigten Monomer, wie Ethylen, Styrol, Vinylestern, Acrylsäure- oder Methacrylsäureestern, die gegebenenfalls mit einem Polyalkylenglykol, wie Polyethylenglykol, gepfropft sind und gegebenenfalls vernetzt sind; den Copolymeren dieses Typs, die in der Kette eine gegebenenfalls N-alkylierte und/oder hydroxyalkylierte Acrylamideinheit enthalten, und den Copolymeren von Acrylsäure und C₁₋₄-Alkylmethacrylat;
C) den Copolymeren, die von Crotonsäure abgeleitet sind, wie den Copolymeren, die in ihrer Kette Vinylacetat- oder Vinylpropionateinheiten und gegebenenfalls weitere Monomere enthalten, wie Allyl- oder Methallylester, Vinylether oder Vinylester einer gesättigten, geradkettigen oder verzweigten Carbonsäure mit langer Kohlenwasserstoffkette, beispielsweise Verbindungen, die mindestens 5 Kohlenstoffatome enthalten, wobei diese Polymere gegebenenfalls gepfropft und vernetzt vorliegen können;
D) den Copolymeren, die von einfach ungesättigten Carbonsäuren oder Carbonsäureanhydriden mit 4 bis 8 Kohlenstoffatomen abgeleitet sind, die ausgewählt sind unter:
- den Copolymeren, die (i) Maleinsäure, Fumarsäure, Itaconsäure oder deren Anhydride und (ii) mindestens ein Monomer enthalten, das unter den Vinylestern, Vinylethern, Vinylhalogeniden, Phenylvinylderivaten und Acrylsäure und ihren Estern ausgewählt ist, wobei die Anhydridfunktionen dieser Polymere gegebenenfalls einfach verestert oder einfach amidiert sind; und
- den Copolymeren, die (i) Maleinsäure, Citraconsäure, Itaconsäure oder deren Anhydride und (ii) ein oder mehrere Monomere enthalten, die unter den Allylestern oder Methallylestern ausgewählt sind, die gegebenenfalls eine oder mehrere Gruppen Acrylamid, Methacrylamid, α-Olefin, Acrylester oder Methacrylester, Acrylsäure oder Methacrylsäure oder Vinylpyrrolidon in ihrer Kette enthalten,
wobei die Anhydridfunktionen dieser Copolymere gegebenenfalls einfach verestert oder amidiert sind; und
(E) den Polyacrylamiden, die Carboxygruppen aufweisen.

14. Zusammensetzung nach dem vorhergehenden Anspruch, **dadurch gekennzeichnet, daß** das anionische fixierende Polymer ausgewählt ist unter:
- den Acrylsäurecopolymeren, beispielsweise dem Acrylsäure/Ethylacrylat/N-*t*-Butylacrylamid-Terpolymer;
- den Copolymeren, die von Crotonsäure abgeleitet sind, beispielsweise den Vinylacetat/Vinyl-*t*-Butylbenzoat/Crotonsäure-Terpolymeren und den Crotonsäure/Vinylacetat/Vinylneododecanoat-Terpolymeren;
- den Polymeren, die von Maleinsäure, Fumarsäure, Itaconsäure oder deren Anhydriden mit Vinylestern, Vinylethern, Vinylhalogeniden, Phenylvinylderivaten und Acrylsäure und ihren Estern abgeleitet sind, beispielsweise den Copolymeren von Methylvinylether und einfach verestertem Maleinsäureanhydrid;
- den Copolymeren von Methacrylsäure und Methylmethacrylat;
- den Copolymeren von Methacrylsäure und Ethylacrylat;
- den Copolymeren von Vinylacetat und Crotonsäure; und
- den Vinylacetat/Crotonsäure/Polyethylenglykol-Terpolymeren.

15. Zusammensetzung nach Anspruch 11, **dadurch gekennzeichnet, daß** das amphotere fixierende Polymer unter den Polymeren ausgewählt ist, die Einheiten aufweisen, welche abgeleitet sind von:
a) mindestens einem Monomer, das unter den Acrylamiden oder Methacrylamiden ausgewählt ist, die am Stickstoffatom mit einer Alkylgruppe substituiert sind,
b) mindestens einem Säurecomonomer, das eine oder mehrere reaktive Carboxygruppen aufweist, und
c) mindestens einem basischen Comonomer, beispielsweise Acrylsäureestern und Methacrylsäureestern mit primären, sekundären oder tertiären Aminosubstituenten und quartären Ammoniumsubstituenten, und dem Quaternisierungsprodukt von Dimethylaminoethylmethacrylat und Dimethylsulfat oder Diethylsulfat.

16. Zusammensetzung nach dem vorhergehenden Anspruch, **dadurch gekennzeichnet, daß** das amphotere fixierende Polymer unter den Copolymeren, die nach CTFA-Nomenklatur als Octylacrylamide/acrylates/butylaminoethylmethacrylate Copolymer bezeichnet werden, und den Methylmethacrlat/Dimethylcarboxymethylammoniumethylmethacrylsäuremethylester-Copolymeren ausgewählt ist.

17. Zusammensetzung nach Anspruch 11, **dadurch gekennzeichnet, daß** das nichtionische fixierende Polymer ausgewählt ist unter:
- den Poly-β-alaninen;
- den Polyalkyloxazolinen;
- den Homopolymeren von Vinylacetat;
- den Copolymeren von Vinylacetat und Acrylestern;
- den Copolymeren von Vinylacetat und Ethylen;
- den Copolymeren von Vinylacetat und Maleinsäureestern;
- den Homopolymeren von Vinylchlorid;
- den Polyethylenwachsen;
- den Polyethylen/Polytetrafluorethylen-Wachsen;
- den Copolymeren von Polyethylen und Maleinsäureanhydrid;
- den Homopolymeren von Alkylacrylaten und den Homopolymeren von Alkylmethacrylaten;
- den Acrylester-Copolymeren, wie beispielsweise den Copolymeren von Alkylacrylaten und Alkylmethacrylaten;
- den Copolymeren von Acrylnitril und einem nichtionischen Monomer, das beispielsweise unter Butadien und den Alkyl(meth)-acrylaten ausgewählt ist;
- den Styrol-Homopolymeren;
- den Copolymeren von Styrol und Alkyl(meth)acrylat;
- den Copolymeren von Styrol, Alkylmethacrylat und Alkylacrylat;
- den Copolymeren von Styrol und Butadien;
- den Copolymeren von Styrol, Butadien und Vinylpyridin; und
- den Copolymeren von Alkylacrylat und Urethan.

18. Zusammensetzung nach Anspruch 11, **dadurch gekennzeichnet, daß** das kationische fixierende Polymer ausgewählt ist unter:
- den Copolymeren von Acrylamid und mit Dimethylsulfat quaternisiertem Dimethylaminoethylmethacrylat,
- den Copolymeren von Acrylamid und Methacryloyloxyethyltrimethylammoniumchlorid,
- den Copolymeren von Acrylamid und Methacryloyloxyethyltrimethylammoniummethosulfat,
- den Copolymeren von Vinylpyrrolidon/Dialkylaminoalkylacrylat oder -methacrylat, die gegebenenfalls quaternisiert sind,
- den Dimethylaminoethylmethacrlat/Vinylcaprolactam/Vinylpyrrolidon-Terpolymeren, und
- dem Copolymer von Vinylpyrrolidon und quaternisiertem Dimethylaminopropylmethacrylamid.

19. Zusammensetzung, die als Aerosol unter Druck steht, **dadurch gekennzeichnet, daß** sie eine Zusammensetzung nach einem der vorhergehenden Ansprüche und mindestens ein Treibmittel enthält.

20. Zusammensetzung nach dem vorhergehenden Anspruch, **dadurch gekennzeichnet, daß** das Treibmittel unter den flüchtigen Kohlenwasserstoffen, wie n-Butan, Propan, Isobutan, chlorierten und/oder fluorierten Kohlenwasserstoffen, Kohlendioxid, Distickstoffoxid, Dimethylether, Stickstoff, Druckluft oder deren Gemischen ausgewählt ist.

21. Zusammensetzung nach einem der Ansprüche 19 oder 20, **dadurch gekennzeichnet, daß** das Treibmittel in Konzentrationen im Bereich von 5 bis 90 Gew.-%, bezogen auf die unter Druck stehende Zusammensetzung, vorliegt.

22. Zusammensetzung nach dem vorhergehenden Anspruch, **dadurch gekennzeichnet, daß** das Treibmittel in Konzentrationen im Bereich von 15 bis 50 Gew.-%, bezogen auf die unter Druck stehende Zusammensetzung, vorliegt.

23. Verfahren zur kosmetischen Behandlung von Keratinsubstanzen, wie dem Haar, das darin besteht, auf die Keratinsubstanzen eine kosmetische Zusammensetzung nach einem der Ansprüche 1 bis 22 aufzubringen.

24. Verwendung der Zusammensetzung nach einem der Ansprüche 1 bis 22 als Lotion für Wasserwellen, Lotion zum Fönen, Zusammensetzung zum Frisieren oder Fixieren der Haare oder zur Herstellung einer solchen Zusammensetzung.

## Claims

1. Cosmetic composition, comprising (a) a fixing polymer, (b) acetone, (c) a C₁-C₄ alcohol, (d) a linear C₅-C₈ hydrocarbon and (e) water **characterized in that** the alcohol concentration is between 5 and 40% by weight relative to the total weight of the composition.

2. Composition according to Claim 1, **characterized in that** the acetone and the linear C₅-C₈ hydrocarbon are present in an acetone/linear C₅-C₈ hydrocarbon ratio of greater than 0.25.

3. Composition according to the preceding claim, **characterized in that** the acetone/linear C₅-C₈ hydrocarbon ratio is between 0.25 and 20.

4. Composition according to any one of the preceding claims, **characterized in that** the fixing polymer is present in concentrations of between 0.05 and 30 % by weight relative to the total weight of the composition.

5. Composition according to the preceding claim, **characterized in that** the fixing polymer is present in concentrations of between 0.5 % and 15 % by weight relative to the total weight of the composition.

6. Composition according to any one of the preceding claims, **characterized in that** the linear C₅-C₈ hydrocarbon concentration is between 1 and 50 % by weight relative to the total weight of the composition.

7. Composition according to the preceding claim, **characterized in that** the linear C₅-C₈ hydrocarbon concentration is between 3 and 25 % by weight relative to the total weight of the composition.

8. Composition according to any one of the preceding claims, **characterized in that** the acetone concentration is between 5 and 80 % by weight, and preferably between 20 and 50 % by weight, relative to the total weight of the composition.

9. Composition according to any one of the preceding claims, **characterized in that** the linear C₅-C₈ hydrocarbons are C₅-C₈ n-alkanes.

10. Composition according to the preceding claim, **characterized in that** the linear C₅-C₈ hydrocarbon is n-pentane.

11. Composition according to any one of the preceding claims, **characterized in that** the fixing polymer is chosen from anionic, cationic, amphoteric, zwitterionic and nonionic polymers and mixtures thereof.

12. Composition according to the preceding claim, **characterized in that** the anionic fixing polymer is chosen from:
- polymers containing carboxylic units derived from unsaturated mono- or dicarboxylic acid monomers of formula: 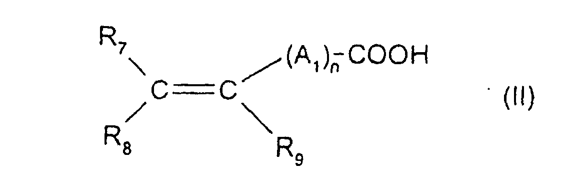 in which n is an integer from 0 to 10, A denotes a methylene group optionally connected to the carbon atom of the unsaturated group or to the neighbouring methylene group when n is greater than 1 via a hetero atom such as oxygen or sulphur, R₇ denotes a hydrogen atom, a phenyl group or a benzyl group, R₈ denotes a hydrogen atom, a lower alkyl group or a carboxyl group and R₉ denotes a hydrogen atom, a lower alkyl group, a
- CH₂-COOH group, a phenyl group or a benzyl group;
- polymers comprising units derived from sulphonic acid, such as vinylsulphonic, styrenesulphonic or acrylamidoalkylsulphonic units.

13. Composition according to the preceding claim, **characterized in that** the anionic fixing polymer is chosen from:
A) homo- or copolymers of acrylic or methacrylic acid or their salts, copolymers of acrylic acid and of acrylamide and their salts, and sodium salts of polyhydroxycarboxylic acids;
B) copolymers of acrylic or methacrylic acids with a monoethylenic monomer such as ethylene, styrene, vinyl esters and esters of acrylic or methacrylic acid, optionally grafted onto a polyalkylene glycol such as polyethylene glycol and optionally crosslinked, copolymers of this type containing an optionally N-alkylated and/or hydroxyalkylated acrylamide unit in their chain, and copolymers of acrylic acid and of C₁-C₄ alkyl methacrylate;
C) copolymers derived from crotonic acid such as those containing in their chain vinyl acetate or propionate units and optionally other monomers such as allylic or methallylic esters, vinyl ether or vinyl ester of a linear or branched saturated carboxylic acid with a long hydrocarbon chain, such as those containing at least 5 carbon atoms, it being possible for these polymers optionally to be grafted and crosslinked;
D) copolymers derived from monounsaturated C₄-C₈ carboxylic acids or anhydrides chosen from:
- copolymers comprising (i) one or more maleic, fumaric or itaconic acids or anhydrides and (ii) at least one monomer chosen from vinyl esters, vinyl ethers, vinyl halides, phenylvinyl derivatives, acrylic acid and its esters, the anhydride functions of these copolymers optionally being monoesterified or monoamidated.
- copolymers comprising (i) one or more maleic, citraconic or itaconic anhydrides and (ii) one or more monomers chosen from allylic or methallylic esters optionally containing one or more acrylamide, methacrylamide, α-olefin, acrylic or methacrylic ester, acrylic or methacrylic acid or vinylpyrrolidone groups in their chain,
the anhydride functions of these copolymers optionally being monoesterified or monoamidated,
E) polyacrylamides containing carboxylate groups.

14. Composition according to the preceding claim, **characterized in that** the anionic fixing polymer is chosen from:
- acrylic acid copolymers such as the acrylic acid/ethyl acrylate/N-tert-butylacrylamide terpolymer;
- copolymers derived from crotonic acid, such as vinyl acetate/vinyl tert-butylbenzoate/crotonic acid terpolymers and crotonic acid/vinyl acetate/vinyl neododecanoate terpolymers;
- polymers derived from maleic, fumaric or itaconic acids or anydrides with vinyl esters, vinyl ethers, vinyl halides, phenylvinyl derivates or acrylic acid and its esters, such as methyl vinyl ether/mono-esterified maleic anhydride copolymers;
- copolymers of methacrylic acid and of methyl methacrylate;
- copolymers of methacrylic acid and of ethyl acrylate;
- vinyl acetate/crotonic acid copolymers;
- vinyl acetate/crotonic acid/polyethylene glycol terpolymers.

15. Composition according to Claim 11, **characterized in that** the amphoteric fixing polymer is chosen from polymers containing units derived from:
a) at least one monomer chosen from acrylamides or methacrylamides substituted on the nitrogen with an alkyl radical,
b) at least one acidic comonomer containing one or more reactive carboxylic groups, and
c) at least one basic comonomer such as esters containing primary, secondary, tertiary and quaternary amine substituents of acrylic and methacrylic acids and the product of quaternization of dimethylaminoethyl methacrylate with dimethyl or diethyl sulphate.

16. Composition according to the preceding claim, **characterized in that** the amphoteric fixing polymer is chosen from copolymers whose CTFA name is octylacrylamide/acrylates/butylaminoethyl methacrylate copolymer and methyl methacrylate/methyl dimethylcarboxymethylammonioethylmethacrylate copolymers.

17. Composition according to Claim 11, **characterized in that** the nonionic fixing polymer is chosen from:
- poly-β-alanines;
- polyalkyloxazolines;
- vinyl acetate homopolymers;
- copolymers of vinyl acetate and of acrylic ester;
- copolymers of vinyl acetate and of ethylene;
- copolymers of vinyl acetate and of maleic ester;
- vinyl chloride homopolymers;
- polyethylene waxes;
- polyethylene/polytetrafluoroethylene waxes;
- copolymers of polyethylene and of maleic anhydride;
- alkyl acrylate homopolymers and alkyl methacrylate homopolymers;
- acrylic ester copolymers such as, for example, copolymers of alkyl acrylates and of alkyl methacrylates;
- copolymers of acrylonitrile and of a nonionic monomer chosen, for example, from butadiene and alkyl (meth)acrylates;
- styrene homopolymers;
- copolymers of styrene and of alkyl (meth)acrylate;
- copolymers of styrene, of alkyl methacrylate and of alkyl acrylate;
- copolymers of styrene and of butadiene;
- copolymers of styrene, of butadiene and of vinylpyridine ;
- copolymers of alkyl acrylate and of urethane.

18. Composition according to Claim 11, **characterized in that** the cationic fixing polymer is chosen from:
- the copolymer of acrylamide and of dimethylaminoethyl methacrylate quaternized with dimethyl sulphate,
- copolymers of acrylamide and of methacryloyloxyethyltrimethylammonium chloride,
- the copolymer of acrylamide and of methacryloyloxyethyltrimethylammonium methosulphate,
- vinylpyrrolidone/dialkylaminoalkyl acrylate or methacrylate copolymers which are or are not quaternized,
- the dimethylaminoethyl methacrylate/vinylcaprolactam/vinylpyrrolidone terpolymer, and
- the quaternized vinylpyrrolidone/dimethylaminopropyl methacrylamide copolymer.

19. Composition pressurized as an aerosol, **characterized in that** it comprises a composition as described in any one of the preceding claims and at least one propellant.

20. Composition according to. the preceding claim, **characterized in that** the propellant is chosen from volatile hydrocarbons such as n-butane, propane, isobutane, chlorohydrocarbons and/or fluorohydrocarbons, carbon dioxide, nitrous oxide, dimethyl ether, nitrogen, compressed air and mixtures thereof.

21. Composition according to either of Claims 19 and 20, **characterized in that** the propellant is present in concentrations of between 5 and 90 % by weight relative to the total weight of the pressurized composition.

22. Composition according to the preceding claim, **characterized in that** the propellant is present in concentrations of between 15 and 50 % by weight relative to the total weight of the pressurized composition.

23. Process for the cosmetic treatment of keratin substances, such as the hair, **characterized in that** it consists in applying a cosmetic composition as defined in any one of Claims 1 to 22 to the keratin substances.

24. Use of the composition as defined in any one of Claims 1 to 22 as, or for the manufacture of, a hair-setting lotion, a blow waving lotion, a hair-styling composition or a hair-fixing composition.
